# EUROPEAN PATENT APPLICATION

(11) **EP 4 023 298 A1**
(43) Date of publication of application: **06.07.2022**
(21) Application number: 20856673.7
(22) Date of filing: 31.08.2020
(51) Int. Cl.: A61P 35/00, A61P 35/04, A61P 37/02, A61P 37/08, C12N 15/09, C12Q 1/6827, A61K 31/423

(54) **PHARMACEUTICAL COMPOSITION FOR CANCER TREATMENT IN PATIENT HAVING SPECIFIC GENE MARKER**

(30) Priority: 30.08.2019 JP 2019158681
(71) Applicant: J-Pharma Co., Ltd., Kanagawa 230-0046 (JP)
(72) Inventor: YOSHITAKE, Masuhiro, Yokohama-shi, Kanagawa 230-0046 (JP); BAMBA, Yoshinori, Yokohama-shi, Kanagawa 230-0046 (JP); ENDOU, Hitoshi, Yokohama-shi, Kanagawa 230-0046 (JP); SUZUKI, Tokiko, Yokohama-shi, Kanagawa 230-0046 (JP)
(74) Representative: TBK
(86) International application number: PCT/JP2020/032828
(87) International publication number: WO 2021/040042

(57) **Abstract**

Provided is a pharmaceutical composition comprising O-(5-amino-2-phenylbenzoxazole-7-yl)methyl-3,5-dichloro-L-tyrosine, or a pharmaceutically acceptable salt thereof, for use in treatment of a cancerous disease in a subject, the pharmaceutical composition being administered to the subject having a Non-Rapid (Slow and/or Intermediate) type NAT2 gene.

## Description

### Technical Field

The present invention relates to a pharmaceutical composition including O-(5-amino-2-phenylbenzoxazole-7-yl)methyl-3,5-dichloro-L-tyrosine, or a pharmaceutically acceptable salt thereof, the pharmaceutical composition is optimally used for a patient with a cancer or the like having a specific genetic marker.

Furthermore, the present invention relates to a method for treating a cancer or the like, wherein the method includes administering a pharmaceutical composition containing O-(5-amino-2-phenylbenzoxazole-7-yl)methyl-3,5-dichloro-L-tyrosine or a pharmaceutically acceptable salt thereof to a patient with a cancer or the like having a specific genetic marker.

### Background Art

An N-acetylation polymorphism was discovered more than 50 years ago, as individual difference in isoniazid neurotoxicity are due to genetic difference in N-acetylation capacity. In addition to isoniazid, many aromatic amine drugs such as sulfamethazine are affected by acetylation polymorphisms, affecting the therapeutic effect and toxicity of many therapeutic agents. N-Acetylation transfer enzyme 2 (NAT2) is an enzyme that transfers the acetyl group of acetyl-CoA to an aromatic amine or hydrazine-containing compound that serves as a substrate for the enzyme. The NAT2 is encoded by the 870-bp gene (NAT2), and the genetic polymorphism in NAT2 is common in a human.

It is known that there are three types of NAT2: a rapid type (Rapid), an intermediate type (Intermediate), and a slow type (Slow) of NAT2 gene acetylation rate (SNP; single nucleotide polymorphism) (Pharmacogenomics, Volume 13, pp. 31-41, 2012). These types can be identified by their gene polymorphisms.

The gene polymorphism in NAT2 is known to modify both the efficacy and toxicity of numerous arylamine and a hydrazine drug and increase the risk for some arylamine carcinogen-related cancers. In addition, a drug called sulfasalazine (SSZ) is acetylated by NAT2, but it is known that a side effect is often likely to occur in a patient with slow acetylation (J Rheumatol. 2002, Dec; 29(12):2492-2499) (Genotyping the NAT2 gene followed by estimation of diplotype configuration before administration of SSZ is likely to reduce the frequency of adverse effects in Japanese patients with RA.http://www.jrheum.org/content/29/12/2492).

Typical NAT2 gene polymorphisms are seven SNPs: (i) 191G>A(rs1801279), (ii) 282C>T(rs1041983), (iii) 341T>C(rs1801280), (iv) 481C>T(rs1799929), (v) 590G>A(rs1799930), (vi) 803A>G(rs1208), and (vii) 857G>A(rs1799931). Among them, the SNPs of (i), (iii), (v), (vi), and (vii) result in a change of amino acid (that is, (i) 191G>A results in the change from arginine to glutamic acid, (iii) 341T>C results in the change from isoleucine to threonine, (v) 590G>A results in the change from arginine to glutamine, (vi) 803A>G results in the change from lysine to arginine, and (vii) 857G>A results in the change from glycine to glutamic acid).

On the other hand, biliary tract cancer has been on the increase in Japan in recent years. The death toll in 2016 was about 18,000, and the cancer is the seventh leading cause of cancer death. For biliary tract cancer, the treatment policy and the effect of chemotherapy differ depending on the site of occurrence. As for chemotherapy, the combination therapy of gemcitabine and cisplatin is currently regarded as standard chemotherapy. However, at present, the recommended secondary therapy for a biliary tract cancer patient who is ineffective with standard chemotherapy is not established, and the clinical need for chemotherapy as secondary therapy is high.

O-(5-amino-2-phenylbenzoxazole-7-yl)methyl-3,5-dichloro-L-tyrosine (hereinafter sometimes referred to as "JPH203") is specifically expressed in cancer cells, and expected to be effective as a new antitumor agent since it has a selective inhibitory effect on type-amino acid transporter 1 (LAT1)(Patent Literature 1).

### Citation List

### Patent Literature

Patent Literature 1: WO 2008/081537 A

### Non-Patent Literature

Non-Patent Literature 1: Pharmacogenomics, Volume 13, pp. 31-41, 2012
Non-Patent Literature 2: Drug Metab Pharmacokinet. 2012; 27(1): pp. 155-61
Non-Patent Literature 3: J. Rheumatol. 2002, Dec; 29(12): 2492-2499
Non-Patent Literature 4: Jutabha et al. Journal of Pharmacological Sciences, Volume 130, Issue 3, Supplement (Abstracts of the 89th Annual Meeting of The Japanese Pharmacological Society) 3-O-84

### Summary of Invention

### Technical Problem

When the inventors are investigating a new secondary chemotherapy for a biliary tract cancer patient who is ineffective with standard chemotherapy, the inventors found that there was a large individual difference in the concentration of an N-acetylated substance in blood and urine compared to the concentration of JPH203 in blood among the subjects who were administered the same dose per body surface area from the results of pharmacokinetic parameter analysis obtained in the domestic phase I study of JPH203. The inventors continued their research and found that there is a relation between the difference in acetylation rate in the gene polymorphism of NAT2 and the safety and drug efficacy of JPH203, and completed the present invention.

### Solution to Problem

That is, the present invention provides a pharmaceutical composition including O-(5-amino-2-phenylbenzoxazole-7-yl)methyl-3,5-dichloro-L-tyrosine, or a pharmaceutically acceptable salt thereof, for use in treatment of a disease in a subject, the pharmaceutical composition being administered to the subject having a Non-Rapid (Slow and/or Intermediate) type NAT2 gene.

The present invention provides a method for treating a disease in a subject, the method including administering to the subject a pharmaceutical composition including O-(5-amino-2-phenylbenzoxazole-7-yl)methyl-3,5-dichloro-L-tyrosine, or a pharmaceutically acceptable salt thereof, after the subject is identified as having a Non-Rapid (Slow and/or Intermediate) type NAT2 gene.

In addition, the present invention provides a use of O-(5-amino-2-phenylbenzoxazole-7-yl)methyl-3,5-dichloro-L-tyrosine or a pharmaceutically acceptable salt thereof to use in the production of a pharmaceutical for the treatment of a disease in a subject, wherein the use is characterized by administering to the subject the pharmaceutical composition including O-(5-amino-2-phenylbenzoxazole-7-yl)methyl-3,5-dichloro-L-tyrosine, or a pharmaceutically acceptable salt thereof, after the subject is identified as having a Non-Rapid (Slow and/or Intermediate) type NAT2 gene.

Furthermore, the present invention provides a method for predicting whether O-(5-amino-2-phenylbenzoxazole-7-yl)methyl-3,5-dichloro-L-tyrosine is effective for a subject with a specific cancer or the like, the method including determining whether or not NAT2 gene in the subject has a Non-Rapid (Slow and/or Intermediate) type NAT2 gene.

The present invention also provides a use of O-(5-amino-2-phenylbenzoxazole-7-yl)methyl-3,5-dichloro-L-tyrosine or pharmaceutically acceptable salt thereof to produce a pharmaceutical for the treatment of a specific disease, the use including determining whether or not NAT2 gene in the subject has a Non-Rapid (Slow and/or Intermediate) type NAT2 gene and predicting whether or not O-(5-amino-2-phenylbenzoxazole-7-yl)methyl-3,5-dichloro-L-tyrosine is effective for the subject of the specific disease before the treatment of the specific disease with the pharmaceutical.

The present invention provides a genetic diagnosis kit for determining whether or not NAT2 gene in the subject has a Non-Rapid type NAT2 gene in order to diagnose whether O-(5-amino-2-phenylbenzoxazole-7-yl)methyl-3,5-dichloro-L-tyrosine is effective for a specific disease, and a pharmaceutical composition including O-(5-amino-2-phenylbenzoxazole-7-yl)methyl-3,5-dichloro-L-tyrosine or a pharmaceutically acceptable salt thereof for use in combination with such a kit or NAT2 genetic diagnosis.

The terms "subject" and "patient" are used interchangeably in the present invention and are preferably human.

### Advantageous Effects of Invention

The pharmaceutical composition of the present invention makes it possible to provide a treatment for a cancer or the like, and the pharmaceutical composition enhances the safety of the drug and improves the efficacy of the effect.

### Brief Description of Drawings

Fig. 1 shows the ratio of Nac-JPH203/JPH203 in rat blood in combination with JPH203 and a NAT2 inhibitor.
Fig. 2 shows the concentration of BPA contained in a pancreatic cancer-derived cell T3M4 after adding boronophenylalanine (BPA) and adding 10 µM of JPH203 15 minutes later.

### Description of Embodiments

Hereinafter, the present invention is described in detail.

### O-(5-amino-2-phenylbenzoxazole-7-yl)methyl-3,5-dichloro-L-tyrosine (JPH203)

An active ingredient of the present invention, O-(5-amino-2-phenylbenzoxazole-7-yl)methyl-3,5-dichloro-L-tyrosine, is a compound disclosed in Patent Literature 1 and is currently undergoing a phase II study by the applicant.

O-(5-amino-2-phenylbenzoxazole-7-yl)methyl-3,5-dichloro-L-tyrosine (JPH203) is metabolized (acetylated) mainly by liver NAT2 after intravenous administration to become an acetylated form (Nac-JPH203), and its physiological activity is greatly reduced (Drug Metab Pharmacokinet. 2012; 27(1): pp. 155-61).

JPH203 can also be used as pharmacologically acceptable salt thereof. For example, in JPH203, the amino group in the molecule forms a salt together with the acid. Such a salt is not particularly limited, and examples thereof include a salt with an inorganic acid such as hydrochloric acid or sulfuric acid, or a carboxylic acid. Among them, a hydrochloride is preferable.

### Pharmaceutical composition

The pharmaceutical composition of the present invention contains JPH203 or a pharmacologically acceptable salt thereof as an active ingredient. In addition, a pharmaceutical additive may be included if necessary. The pharmaceutical composition of the present invention can be orally administered in the form of a solid preparation such as a tablet, a granule, a fine granule, a powder or a capsule, or a liquid, a jelly, a syrup and the like. Alternatively, the pharmaceutical composition drug may be administered parenterally in the form of an injection, a nasal agent, a suppository, an inhalant, a transdermal agent and the like.

In particular, since the pharmaceutical composition of the present invention can be used as an anticancer agent or the like, it is preferably formulated as the injection. Examples of such an injection include the injection containing the active ingredient of the present invention, a pH adjuster, and cyclodextrins.

Specific examples of the injection include intravenous, subcutaneous, intradermal, intramuscular injection, and intravenous drip infusion.

Examples of the pH adjuster that can be blended in the injection of the present invention include alkali metal hydroxides such as sodium carbonate, potassium carbonate, sodium hydroxide and potassium hydroxide, alkali metal hydrides such as sodium hydride and potassium hydride, and alkali metal or alkaline earth metal carbonate, and sodium hydroxide and sodium hydroxide are particularly preferable.

The injection can be appropriately adjusted to an appropriate pH using the pH adjuster. The pH of the injection according to the present embodiment is preferably 3 to 6, more preferably 3 to 5, further preferably 3 to 4.5, and particularly preferably 3.5 to 4.5.

Examples of cyclodextrins that can be blended in the injection of the present invention include an unmodified cyclodextrin and a modified cyclodextrin. Examples of the unmodified cyclodextrin include α-cyclodextrin, β-cyclodextrin, and γ-cyclodextrin. Furthermore, Examples of the modified cyclodextrin include dimethyl-α-cyclodextrin, dimethyl-β-cyclodextrin, dimethyl-γ-cyclodextrin, hydroxypropyl-α-cyclodextrin, hydroxypropyl-β-cyclodextrin, hydroxypropyl-γ-Cyclodextrin, sulfobutylether-α-cyclodextrin, sulfobutylether-β-cyclodextrin, sulfobutylether-γ-cyclodextrin, and maltosyl-α-cyclodextrin.

As for cyclodextrins, one kind may be used alone, or two or more kinds may be used in any combination. Cyclodextrins are hydroxypropyl-β-cyclodextrin or sulfobutylether-β-cyclodextrin is preferable, and sulfobutylether-β-cyclodextrin is more preferable from the viewpoint of reducing the number of insoluble fine particles formed even when dissolved in a non-strongly acidic aqueous solution and improving the resolubility of the lyophilized preparation in a non-strongly acidic aqueous solution.

The sulfobutylether cyclodextrin has the structure shown in the following Formula 1, and the inside of the cyclic structure is highly hydrophobic. Therefore, it forms a complex with O-(5-amino-2-phenylbenzoxazole-7-yl)methyl-3,5-dichloro-L-tyrosine, which is also highly hydrophobic, by hydrophobic interaction. The reference to "sulfobutylether-cyclodextrin complex" in the present description refers to the above-mentioned hydrophobic interaction.

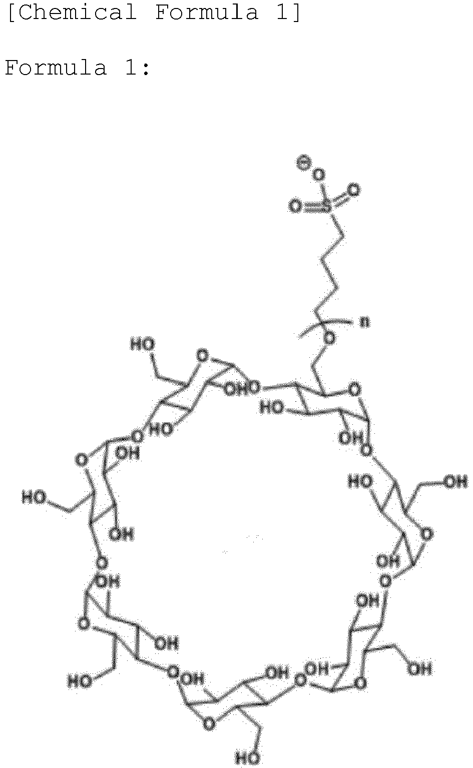

Alternatively, the active ingredient, O-(5-amino-2-phenylbenzoxazole-7-yl)methyl-3,5-dichloro-L-tyrosine, may be used in the injection as sulfobutylether-cyclodextrin complex (hereafter referred to as "JPH203-SBECD") thereof.

If necessary, a buffer, a suspending agent, a solubilizing agent, a stabilizer, an isotonic agent, a preservative and the like may be added to the injection according to the present invention.

Examples of the buffer include a borate buffer, a phosphate buffer, a citric acid buffer, an acetate buffer, and a Tris buffer.

Examples of the suspending agent include methyl cellulose, polysorbate 80, hydroxyethyl cellulose, gum arabic, tragant powder, sodium carboxymethyl cellulose, polyoxyethylene sorbitan monolaurate, poloxamer, hydroxypropyl methyl cellulose (HPMC), and sodium alginate.

Examples of the solubilizing agent include polyoxyethylene hydrogenated castor oil, polysorbate 80, nicotinic acid amide, polyoxyethylene sorbitan monolaurate, macrogol, glycerin fatty acid ester, lipoaminoacid, polyethylene glycol and the like.

Examples of the stabilizer include sodium sulfite, sodium metasulfite and the like, tonicity agents include glycerin, and sodium chloride. Examples of the isotonic agent include methyl paraoxybenzoate, ethyl paraoxybenzoate, and sorbic acid.

When the lyophilized preparation is dissolved in water, the pH is preferably 3 to 6, more preferably 3 to 5, further preferably 3 to 4.5, and particularly preferably 3.5 to 4.5.

The lyophilized preparation can be produced by a conventionally known method for producing a lyophilized preparation. Examples of the method include a method of drying while keeping the vacuum degree at about 20 Pa or less and raising the temperature until it reaches room temperature, after freezing at a temperature of -25°C or lower.

The injection according to the present invention may be the lyophilized preparation. Such a lyophilized preparation can be used as a before use type injection by dissolving it in, for example, one kind or two or more kinds of solvents of a distilled water for injection, an infusion solution, and electrolytic solution before use.

The pharmaceutical composition of the present invention can be used for the treatment of a specific disease, and example of the target diseases include carcinomas such as fibroma, lipoma, myxoma, chondroma, osteoma, hemangiomas, hemangioendothelioma, lymphoma, myeloma, myeloid sarcoma, reticular tumor, reticular sarcoma, melanoma, myoma, neuroma, glioma, schwannoma, sarcoma, osteosarcoma, muscle type, fibrosarcoma, papilloma, adenoma, cyst, brain tumor, cervical cancer, tongue cancer, pharyngeal cancer, laryngeal cancer, thyroid cancer, esophageal cancer, lung cancer, breast cancer, stomach cancer, small intestine cancer such as duodenum, jejunum, and ileum, colon cancer such as colon, cecum, rectum, bladder cancer, renal cancer, bile sac cancer, prostate cancer, uterine body cancer, cervical cancer, ovarian cancer, mesothelioma, head and neck cancer, adrenal cancer, gastrointestinal stromal tumor, appendiceal cancer, biliary tract cancer, pancreatic cancer, liver cancer, skin cancer, villous cancer, head and neck cancer, metastatic cancer, invasive cancer, or the like; and a mixed tumor, a metastatic tumor, or the like thereof.

Among these, biliary tract cancer, colon cancer, pancreatic cancer, esophageal cancer, breast cancer, lung cancer, prostate cancer, bladder cancer, brain tumor, stomach cancer, liver cancer, skin cancer, chorionic villi cancer, kidney cancer, head and neck cancer, tongue cancer, metastatic cancer, invasive cancer, or the like are preferable. Furthermore, biliary tract cancer, colon cancer, pancreatic cancer, esophageal cancer, and breast cancer are recommended.

### Dosing regimen

A dosing regimen to which the pharmaceutical composition of the present invention is applied is selected according to various factors such as patient type, race, age, body weight, sex, and medical condition; severity of condition to be treated; route of administration; and patient's liver and renal function. Physician can easily determine and prescribe the effective amount of a drug needed to prevent, prevent or stop the progression of the symptom.

In the pharmaceutical composition of the present invention, the dose of the active ingredient can be appropriately selected according to the degree of symptoms, the age, sex, body weight, sensitivity difference, timing of administration, administration interval, etc. of the patient, and from the viewpoint of efficacy and safety, 1 mg/m² to 60 mg/m² (body surface area) is exemplified once, preferably 12.5 mg/m² to 60 mg/m², 12.5 mg/m² to 25 mg/m², or 10 mg/m² to 40 mg/m² (surface area) is exemplified, and 25 mg/m² is particularly recommended. The dose may be reduced to 12.5 mg/m² or the like depending on the symptoms.

Example of the administration regimen of the pharmaceutical composition of the present invention include
- the pharmaceutical composition is administered as one cycle with a drug withdrawal for a certain period following continuous administration for a certain period,
- the pharmaceutical composition is administered as one cycle of a total of 14 days with the drug withdrawal for 9 days following continuous administration for 5 days,
- a certain amount of the pharmaceutical composition is continuously administered intravenously over a certain period of time,
- 100 mL of the pharmaceutical composition is continuously administered intravenously over 90 minutes,
- the pharmaceutical composition administered to the patient at a dose of 1 to 60 mg/m²,
- the pharmaceutical composition is administered to the patient at a dose of 12.5 to 60 mg/m²,
- the pharmaceutical composition is administered to the patient at a dose of 12.5 to 25 mg/m²,
- the pharmaceutical composition is administered to the patient at a dose of 25 mg/m²,
   and these can be applied in combination as needed.

The pharmaceutical composition of the present invention can be highly effective when administered to a patient with a cancer or the like having a Non-Rapid (Slow and/or Intermediate) type (that is, the type with slow acetylation by NAT2) NAT2 gene. In the present specification, the "Non-Rapid type NAT2 gene" means an intermediate type and a slow type phenotype among the three phenotypes of the rapid type (Rapid), the intermediate type (Intermediate), and the slow type (Slow) of NAT2 gene acetylation rate.

When the phenotype of NAT2 acetylation is analyzed by the combination of 2-SNP, 3-SNP, and 4-SNP (Table 2-1) for the seven SNPs (481C>T, 282C>T, 857G>A, 803A>G, 341T>C, 590G>A, 191G>A) representing the gene polymorphism of NAT2, the phenotype composed of homozygote of NAT2*4, which is an SNP-free (standard) haplotype, is considered to be the type with a rapid acetylation rate (Rapid), heterozygote mutated in any one of the haplotypes is considered to be the type with an intermediate acetylation rate (Intermediate), homozygote of all mutant haplotypes or a combination of two or more mutant haplotype heterozygotes is considered to be the type with a slow acetylation rate (Slow) (Pharmacogenomics, Vol. 13, pp. 31-41, 2012). Here, the "NAT2*4" allele means an SNP-free haplotype. In addition, according to Pharmacogenomics, Vol. 13, pp. 31-41, 2012, regarding the Rapid type, in addition to "NAT2*4", the patient with "NAT2*11, NAT2*12, NAT2*13" that do not cause amino acid mutations are also classified as the Rapid type.

Specifically, the NAT2 acetylation factor phenotype inferred by the two SNPs is determined by the analysis of the two SNPs: rs1041983 (282C>T) and rs1801280 (341T>C). The NAT2 acetylation factor phenotype inferred by the three SNPs is determined by the analysis of the three SNPs: rs1799929 (481C>T), rs1799930 (590G>A), and rs1799931 (857G>A). The NAT2 acetylation phenotype inferred by the four SNPs is determined by analysis of the four SNPs: rs1801279 (191G>A), rs1801280 (341T>C), rs1799930 (590G>A), and rs1799931 (857G>A).

When JPH203 is acetylated by NAT2, the inhibitory activity on the cancer cell decreases. However, if the patient predominantly has a NAT2 gene phenotype that is less susceptible to acetylation, that is, in the NAT2 gene of the patient with a cancer or the like, if the patient has the intermediate type (Intermediate) and slow type (Slow) phenotypes (Non-Rapid type NAT2 gene), since the acetylation rate of JPH203 is slowed down, JPH203 is difficult to be acetylated and the activity of JPH203 lasts for a long time.

The analysis of the NAT2 gene polymorphism in the present invention can be performed by a conventionally known method. For example, it can be performed by extracting DNA from the blood of a subject, processing the DNA by the microarray method, then reading the genotype and performing a test, and then performing data analysis.

Therefore, for the purpose of predicting the safety and efficacy of a cancer therapeutic agent and providing an appropriate therapeutic agent to a patient with a cancer or the like, administration of the pharmaceutical composition of the present invention to a cancer patient, for example, appropriately performed as follows.

Blood is drawn from the human patient with or suspected of having a cancer, DNA is extracted from the blood sample, and the NAT2 gene polymorphism is confirmed. For the patient in which the NAT2 gene polymorphism was determined to have a Non-Rapid type (that is, determined to be an intermediate type of acetylation rate (Intermediate) or a slow acetylation type of acetylation rate (Slow)), 100 mL of O-(5-amino-2-phenylbenzoxazole-7-yl)methyl-3,5-dichloro-L-tyrosine sulfobutylether-cyclodextrin complex is continuously administered intravenously over 90 minutes at a concentration of 25 mg/m² to the subject. At the time, the pharmaceutical composition is administered as one cycle of a total of 14 days with the drug withdrawal for 9 days following continuous administration for 5 days.

Then, in the NAT2 genetic diagnosis of the patient, it is possible to predict whether or not O-(5-amino-2-phenylbenzoxazole-7-yl)methyl-3,5-dichloro-L-tyrosine is effective for the subject with specific diseases such as a cancer by determining whether or not the patient has the Non-Rapid type NAT2 gene.

The therapeutic effect on a cancer or the like may be better in animal models and animal species in which the metabolic capacity of NAT2 is suppressed, as compared with the animal model in which the metabolic capacity of NAT2 is normal. This is also applicable to a human.

Furthermore, it is possible to provide an excellent treatment for a specific disease, particularly a cancer or the like, which enhance the safety of the drug and improve the efficacy of the effect, by applying the administration method for the pharmaceutical composition of the present invention to a patient.

In addition, as a further embodiment of the present invention, a pharmaceutical composition including O-(5-amino-2-phenylbenzoxazole-7-yl)methyl-3,5-dichloro-L-tyrosine or the pharmaceutically acceptable salt thereof, which is used in combination with NAT2 genetic diagnosis, is provided. When the pharmaceutical composition is used for the treatment of a specific disease, particularly a cancer or the like, its effect can be enhanced.

As described above, the NAT2 genetic diagnosis tests for the NAT2 gene polymorphism to determine whether or not NAT2 gene has a Non-Rapid type NAT2 gene. The NAT2 genetic diagnosis can be performed by a conventionally known diagnostic method, for example, the analysis method of the NAT2 gene polymorphism described later. Alternatively, NAT2 genetic diagnosis can use the service of inspection institutions such as a company that provides genetic testing and genetic diagnosis.

Alternatively, the NAT2 genetic diagnosis can be performed by the NAT2 genetic diagnosis kit. The NAT2 genetic diagnosis kit is a collection of a sample collection instrument and a reagent necessary for carrying out the analysis method of the NAT2 gene polymorphism described later. Examples of the reagent provided in the kit include a PCR enzyme solution, a primer/probe, dH₂O, positive control DNA, and a dedicated buffer for control dilution.

In another embodiment, a pharmaceutical composition of the invention including O-(5-amino-2-phenylbenzoxazole-7-yl)methyl-3,5-dichloro-L-tyrosine or a pharmaceutically acceptable salt thereof is used in combination with the NAT2 genetic diagnosis or the genetic diagnosis kit. When these are used in combination, it is possible to enhance the effect of O-(5-amino-2-phenylbenzoxazole-7-yl)methyl-3,5-dichloro-L-tyrosine on a specific disease, especially when cancer is treated.

As a further embodiment, provided is an effective method for treating a specific disease, for example, a cancer or the like, by using a pharmaceutical composition containing O-(5-amino-2-phenylbenzoxazole-7-yl)methyl-3,5-dichloro-L-tyrosine or a pharmaceutically acceptable salt thereof of the present invention in combination with a NAT2 genetic diagnosis or a genetic diagnosis kit.

For example, the pharmaceutical compositions including O-(5-amino-2-phenylbenzoxazole-7-yl)methyl-3,5-dichloro-L-tyrosine or a pharmaceutically acceptable salt thereof can be administered to a subject suffering from a cancer in the following administration regimen in combination with the measurement of the NAT2 gene polymorphism.

(1) The subject having the Non-Rapid (Slow and/or Intermediate) type NAT2 gene is identified and selected;
(2) The pharmaceutical composition is administered to the subject identified as having a Non-Rapid (Slow and/or Intermediate) type NAT2 gene.

Furthermore, a pharmaceutical composition comprising O-(5-amino-2-phenylbenzoxazole-7-yl)methyl-3,5-dichloro-L-tyrosine or a pharmaceutically acceptable salt thereof of the present invention can be used in combination with a NAT2 inhibitor for the treatment of a disease such as a cancer. Examples of the NAT2 inhibitor include acetaminophen.

JPH203 is rapidly N-acetylated by NAT2 in the hepatic cytosol to become Nac-JPH203, and Nac-JPH203 is known to have lower selectivity and activity for LAT1 than JPH203, and when used in combination with the NAT2 inhibitor, it suppresses the acetylation of JPH203 and maintains the effect of JPH203.

When the amount of O-(5-amino-2-phenylbenzoxazole-7-yl)methyl-3,5-dichloro-L-tyrosine or pharmaceutically acceptable salt thereof and the NAT2 inhibitor to be used, the respective effective amounts are used and can be appropriately selected depending on the administration subject, administration route, disease, combination and the like.

The time of administration of O-(5-amino-2-phenylbenzoxazole-7-yl)methyl-3,5-dichloro-L-tyrosine or the pharmaceutically acceptable salt thereof and the NAT2 inhibitor is not particularly limited, and O-(5-amino-2-phenylbenzoxazole-7-yl)methyl-3,5-dichloro-L-tyrosine or the pharmaceutically acceptable salt of the present invention and the NAT2 inhibitor may be administered to the administration subject at the same time or at different times.

When O-(5-amino-2-phenylbenzoxazole-7-yl)methyl-3,5-dichloro-L-tyrosine or a pharmaceutically acceptable salt thereof is used in combination with a NAT2 inhibitor, the respective doses can be reduced, the treatment period can be set longer, and synergistic effects can be obtained.

### Combined use with BNCT (Boron Neutron Capture Therapy)

Furthermore, O-(5-amino-2-phenylbenzoxazole-7-yl)methyl-3,5-dichloro-L-tyrosine or a pharmaceutically acceptable salt thereof of the present invention can be used in combination with a boron neutron capture therapy for the treatment of a cancer.

Examples of the compound used for the boron neutron capture therapy include a boronated amino acid, and in particular, boronophenylalanine (BPA) (trade name: Borofalan B10) is recommended.

The boron neutron capture therapy uses the boronated amino acid in combination with neutron ray irradiation. In the method, a boronated amino acid (for example, boronophenylalanine BPA) is injected into the body and taken up by a cancer cell. At this time, BPA is taken up by the cancer cell through LAT1. When a neutron ray is irradiated there, nuclear fission occurs in the cancer cell and cell destruction occurs. However, since BPA is easily excreted from the cancer cell through LAT1, a high dose of BPA is required. On the other hand, BPA is also taken up by a normal cell when administered in a high dose. As a result, BPA affects surrounding normal cells, causes a side effect, and also affects a kidney of an organ that metabolizes and excretes.

Therefore, when O-(5-amino-2-phenylbenzoxazole-7-yl)methyl-3,5-dichloro-L-tyrosine of the present invention is used in combination with the boron neutron capture therapy, it is possible to efficiently contain BPA in the cancer cell, thereby reducing the BPA dose and reducing the effect (= side effect) on the normal cell (Jutabha et al., Journal of Pharmacological Sciences, Volume 130, Issue 3, Supplement (89th Japan) Abstracts of the Annual Meeting of the Japanese Pharmacological Society) 3-O-84).

That is, as described in the literature of Jutabha et al., BPA is taken up into a cell through LAT1 on the surface of a cancer cell. As it is, BPA is also excreted extracellularly by LAT1. LAT1 can be covered and BPA can be contained inside a cancer cell by administering JPH203 after BPA is taken up by a cancer cell and before it is excreted from the cancer cell (the time difference between BPA and JPH203 administration is important). Therefore, a cancer cell can be efficiently attacked even if the dose of BPA is small. That is, JPH203 is effective in containing BPA. Since Nac-JPH203 has a lower cell proliferation inhibitory effect than JPH203, it is considered that the NAT2 non-Rapid type is more effective than the NAT2 Rapid type.

The current standard BPA administration method is intravenous infusion, which is administered at a rate of 200 mg/kg/h for 2 hours before neutron irradiation, and an amount of 100 mg/kg is administered at a constant rate of about 100 mg/kg/h according to the irradiation end time, during irradiation. If it is too conscious to keep the BPA in cancer cells at a high concentration as a goal setting, the concentration of extracellular matrix increases and the effect on a normal cell also increases. As for the timing of administration of JPH203, after intravenous infusion of BPA at 100 to 200 mg/kg/h for 30 minutes to 1 hour was completed before neutron irradiation, intravenous infusion of JPH203 was immediately performed at a rate of 10 to 25 mg/kg/h. The neutron ray is irradiated 15 to 30 minutes after the start of JPH203 administration.

BPA is also taken up into a normal cell through LAT2 on the surface of the normal cell when the local concentration is high, but the uptake efficiency of LAT2 is lower than that of LAT1. Since JPH203 covers only LAT1, BPA inside a cancer cell continues to be contained at a sufficiently high concentration at the start of neutron irradiation.

When O-(5-amino-2-phenylbenzoxazole-7-yl)methyl-3,5-dichloro-L-tyrosine of the present invention or a pharmaceutical composition containing the same is used in combination with the boron neutron capture therapy, the following administration regimen is recommended.

For example, BPA is intravenously infused to a cancer patient at a rate of 200 mg/kg/h for 1 hour. At this point, BPA administration is discontinued, intravenous administration of JPH203 at 10 to 20 mg/kg/h is started, neutron ray irradiation is started 30 minutes later, and intravenous infusion of JPH203 is continued until the end of irradiation during irradiation.

Furthermore, the combination of O-(5-amino-2-phenylbenzoxazole-7-yl)methyl-3,5-dichloro-L-tyrosine of the present invention and the boron neutron capture therapy may be combined with the method for measuring NAT2 gene polymorphism.

Such a method includes the following dosing regimens;
(1) The subject having the Non-Rapid (Slow and/or Intermediate) type NAT2 gene is identified and selected;
(2) a boron-containing compound are administered to the subject identified as having a Non-Rapid (Slow and/or Intermediate) type NAT2 gene prior to administration of the pharmaceutical composition; and
(3) the pharmaceutical composition is administered to the subject.

### Examples

Hereinafter, the present invention is described with reference to specific embodiments, but the present invention is not limited to the embodiments, and it is understood that various changes and modifications thereof may be made by those skilled in the art without departing from the scope or intent of the invention as set forth in the appended claims.

### Example 1

Drug used: JPH203-SBECD (50 mg as an active ingredient) is dissolved in 9.7 ml of water for injection to a concentration of 50 mg/10 ml, and finally the total volume is 100 ml according to the body surface area of a patient.

An uncontrolled, open-label, domestic phase I study was conducted to evaluate the safety of JPH203-SBECD (12, 25, 40, 60, 85 mg/m²) and determine the recommended dose in the next phase, and analyze the pharmacokinetics for patients with an advanced-stage solid cancer who are ineffective or intolerant to a standard treatment. The initial dose of the investigational drug was 12 mg/m², and 5 levels (12, 25, 40, 60, 85 mg/m²) were set to increase the dose according to the modified Fibonacci method. Three subjects enrolled within 28 days of obtaining consent were targeted for in each level, and a single administration was administered within 4 days after enrollment. After the first subject received a single administration, when the results of the pre-dose examination on the first day of repeated administration (cycle 1) for the first 7 days confirmed that there was no problem with safety, administration of the second and subsequent subjects was started.

The cycle of repeated administration of the investigational drug was continued from 7 days after the single administration to the discontinuation criteria for the subjects who received the single administration and could be judged that there was no problem in their health condition. In addition, when it was confirmed that there was no problem with safety in the first subject 49.5 hours after the start of administration on the seventh day of the cycle 1, the administration of the second and subsequent subjects was started.

The subject received the investigational drug, which is prepared to achieve the desired dose when the total volume is 100 mL of an appropriate infusion solution such as physiological saline or Ringer's solution, intravenously continuously over 90 minutes through an infusion circuit containing a final filter (pore size: 0.22 µm) and a metering pump in single administration and cycle 1 (once daily, 7 days). In the same manner after cycle 2, the subjects received the investigational drug, which is prepared with an appropriate infusion solution such as physiological saline and Ringer's solution so as to achieve the desired concentration, once daily for 7 days, at the same dosage and dose as cycle 1.

As a result, partial response (PR): 1 patient and stable (SD): 2 patients (total response rate 20.0%, disease control rate 60.0%) were observed in 5 patients with the biliary tract cancer, the efficacy of the drug in patients with the biliary tract cancer has been suggested. There were no clinically problematic adverse events or side effects in terms of safety. From these results, it was considered that there was no problem with tolerability and a certain degree of efficacy could be expected for the patient with the biliary tract cancer.

From the results of pharmacokinetic parameter analysis obtained in the domestic phase I study, it was confirmed that there is a large individual difference in the concentration of an N-acetylated substance (Nac-JPH203) in blood and urine among the subjects who received the same dose per body surface area. JPH203 is taken up into a hepatocyte by the organic anion transporter (OATP), which is a hepatic transporter, after intravenous administration. It was considered that JPH203 taken up into the hepatocyte was converted to Nac-JPH203 by NAT2 in the hepatocyte and excreted in bile by a bile excretion transporter. Therefore, it was speculated that the individual difference in the concentration of Nac-JPH203 in urine were due to the difference in the acetylation rate of NAT2, which is considered to be responsible for most of the metabolism of JPH203 in vivo.

### Pharmacokinetics analysis

Blood (whole blood) and urine were collected from the subjects. The whole blood used for the concentration measurement was collected 0.5, 1, 1.5, 2, 3, 4.5, 6, 12, 25.5, and 49.5 hours after the start of administration. The urine used for the concentration measurement was collected by collecting urine every 0 to 3, 3 to 6, 6 to 12, 12 to 25.5 hours from the start of administration. The JHP203 concentration and the Nac-JPH203, which is an acetyl form thereof, concentration in blood and urine, were measured using a validated LC-MS/MS measurement system.

Pharmacokinetic parameters were calculated using the JPH203 and Nac-JPH203 concentrations in blood. Among them, AUC0-∞ was used to determine the ratio of Nac-JPH203 to JPH203 in the blood of each subject. For each subject, the total mass of JPH203 and Nac-JPH203 contained in the urine collected from the start of administration to 25.5 hours was calculated, and the ratio of Nac-JPH203 to JPH203 in the urine in each subject was calculated.

### Measurement of NAT2 gene polymorphism

The measurement of NAT2 gene polymorphism can be carried out by either the following real-time PCR method or the method using the Luminex System. In either method, the following seven types of SNPs are analyzed for NAT2.

**(Table 1)**

| No. | Genomic DNA | rs1801279 | rs1041983 | rs1801280 | rs1799929 | rs1799930 | rs1208 | rs1799931 |
|---|---|---|---|---|---|---|---|---|
| 1 | NA06985 | G/G | C/C | C/C | T/T | G/G | G/G | G/G |
| 2 | NA07056 | G/G | T/T | T/T | C/C | A/A | A/A | G/G |
| 3 | NA18939 | G/G | C/T | T/T | C/C | G/A | A/A | G/G |
| 4 | NA18941 | G/G | C/T | T/T | C/C | G/G | A/A | G/A |
| 5 | NA18948 | G/G | C/C | T/T | C/C | G/G | A/A | G/G |
| 6 | NA18959 | G/G | T/T | T/T | C/C | G/A | A/A | G/A |
| 7 | NA18974 | G/G | T/T | T/T | C/C | G/G | A/A | A/A |
| 8 | NA19025 | G/G | C/C | T/C | C/T | G/G | G/A | G/G |
| 9 | NA19456 | G/A | C/C | T/C | C/T | G/G | G/A | G/G |
| 10 | NA19835 | A/A | T/T | T/T | C/C | G/G | A/A | G/G |

### Real-time PCR method

The measurement is performed by the real-time PCR method according to the following method. The details of the protocol can be changed as appropriate.
(i) After measuring the DNA concentration of NAT2, two types of Forward Primer and one type of Reverse Primer that can amplify the region containing 7 types of SNPs to be analyzed is designed.
(ii) The PCR conditions are examined using these PCR primers, and the final PCR primer set and PCR conditions to be used are determined. Then, according to the determined conditions, the genomic DNA is subjected to sequence analysis by the Sanger sequencing method.
(iii) After PCR, the PCR product is purified and measured by a capillary sequencer. The PCR primer is used as the sequencing primer, and sequencing is performed from both sides of the PCR product.

### Method using Luminex System

The measurement is performed by the Luminex System method according to the following method. The details of the protocol can be changed as appropriate.
(i) A probe solid-phase bead corresponding to 7SNPs to be analyzed for NAT2 is created.
(ii) The NAT2 gene is amplified with a biotin-labeled primer. The PCR amplification product is bound to a probe and fluorescently labeled with SA-PE.
(iii) The data is acquired by the Luminex System and a type of NAT2 gene is displayed by an analysis software.

### Analysis of NAT2 gene polymorphism

The NAT2 gene polymorphism test was performed as follows. DNA was extracted from the blood sample at the time of the phase I study, and the phenotype was classified from the information of each SNP according to the gene polymorphism of NAT2. The outline of the method is as follows.

DNA was extracted using a QIAamp DNA Blood Mini kit (registered trademark) (QIAGEN K.K.) after eluting 200 µL of whole blood into 100 µL of AE buffer. An ultra-trace spectrophotometer (Nano Drop 2000 (registered trademark), Thermo Fisher Scientific) was used to measure an array and a haplotype associated with SNPs at the site encoding NAT2 (the concentration wad adjusted to 5 ng/µL). For gene polymorphism analysis, TaqMan (registered trademark) SNP Genotyping assay was used (reaction volume: 5 µL). The PCR primer and fluorescent probe required to detect the SNP were designed using Primer Express (trademark) (Applied Biosystems, CA, USA). The fluorescent probe is labeled with s reporter dye (carboxyfluorescein or VIC (registered trademark), Applied Biosystems) and was made to react specifically to one of the two bases in the seven NAT2SNPs (rs1801279(191G>A), rs1041983(282C>T), rs1801280(341T>C), rs1799929(481C>T), rs1799930(590G>A), rs1208(803A>G), rs1799931 (857G>A)). The PCR reaction conditions were 10 minutes at 95°C, followed by 50 cycles of 15 seconds at 92°C and 90 seconds at 60°C.

For seven SNPs (481C>T, 282C>T, 857G>A, 803A>G, 341T>C, 590G>A, and 191G>A) representing the NAT2 gene polymorphisms, the genes of 16 subjects who participated in the Phase I study were analyzed.

Analysis of the NAT2 acetylation phenotype by a combination of 2-SNP, 3-SNP, and 4-SNP (Table 2-1) shows that all of the types with a rapid acetylation rate (Rapid) are composed of the homozygote of NAT2*4 which is standard haplotypes, the types with an intermediate acetylation rate (Intermediate) are composed of the heterozygotes mutated in any one of the haplotypes, and all of the types with a slow acetylation rate (Slow) are composed of homozygotes of all mutant haplotypes or combinations of two or more mutant haplotype heterozygotes (Table 2-2).

**(Table 2-1)**

| | Referred SNPs |
|---|---|
| NAT2 acetylation phenotype inferred 2-SNP | 282C>T and 341T>C |
| NAT2 acetylation phenotype inferred 3-SNP | 481C>T, 590G>A, and 857G>A |
| NAT2 acetylation phenotype inferred 4-SNP | 191G>A, 341T>G, 590G>A, and 857G>A |

| | |
|---|---|
| Phenotype classification by NAT2 genotyping | |

**(Table 2-2)**

| Acetylation category (NAT2) | SNPs |
|---|---|
| Rapid acetylation phenotype (Rapid) | 2*4 (standard haplotype) homozygotes for all SNPs |
| Intermediate acetylation phenotype (Intermediate) | Heterozygotes mutated in any one of the haplotypes |
| Slow acetylation phenotype (Slow) | Homozygotes of all mutant haplotypes or combinations of two or more mutant haplotype heterozygotes |

### Phenotype classification by NAT2 genotyping

The NAT2 SNP phenotypes of 16 subjects were consistent with any combination of 2-SNP, 3-SNP, and 4-SNP, regardless of a cancer type (colon cancer, biliary tract cancer, pancreatic cancer, esophageal cancer, and breast cancer). Table 3 shows the results in detail.

**(Table 3)**

| Patient ID | Type of cancer | Two SNPs | Three SNPs | Four SNPs | Clinical results |
|---|---|---|---|---|---|
| 102 | PAAD | R | R | R | PD |
| 103 | CHOL | S | S | S | PR |
| 104 | COAD | R | R | R | SD |
| 201 | COAD | R | R | R | PD |
| 202 | CHOL | S | S | S | SD |
| 203 | CHOL | R | R | R | PD |
| 301 | PAAD | I | I | I | PD |
| 302 | BRCA | R | R | R | PD |
| 303 | COAD | S | S | S | SD |
| 401 | ESCA | R | R | R | PD |
| 402 | COAD | S | S | S | PD |
| 403 | COAD | R | R | R | PD |
| 404 | CHOL | S | S | S | PD |
| 405 | COAD | I | I | I | PD |
| 406 | CHOL | I | I | I | SD |
| 501 | PAAD | R | R | R | PD |

| | | | | | |
|---|---|---|---|---|---|
| Three types of NAT2: Phenotype classification by SNP genotyping | | | | | |

SD: Stable (tumor size does not change)
PD: Partial response (the sum of tumor sizes increased by 20% or more and the absolute value increased by 5 mm or more, or a new lesion appeared)
PAAD: Pancreatic cancer (Pancreas Adenocarcinoma)
CHOL: Biliary tract cancer (Cholangiocarcinoma)
COAD: Colon cancer (Colon Adenocarcinoma)
BRCA: Breast Cancer (Breast Adenocarcinoma)
ESCA: Esophageal cancer (Esophageal Carcinoma)

### Relation between NAT2 phenotype and clinical results (safety and efficacy)

When the variation in the Nac-JPH203/JPH203 concentration ratio of 16 individuals is classified into NAT2 Rapid type, Intermediate type, and Slow type, the type with a large amount of the metabolite Nac-JPH203 is NAT2 Rapid type, and conversely, the individual with the low metabolite was of the NAT2 Slow type. Among these NAT2 Rapid groups, 4 subjects with a high Nac-JPH203 ratio showed an increase in liver function test values, and 2 subjects were judged to have DLT (Dose Limiting Toxicity). These patients included 2 patients of colon cancer, 1 patient of esophageal cancer, and 1 patient of pancreatic cancer, and did not include a patient with the biliary tract cancer.

That is, in the cancer patient with NAT2 Rapid type, the value of liver dysfunction due to JPH203 administration was high.

When the clinical efficacy of the JPH203-administered patient was evaluated by DCR (Disease Control Rate), the number of patients who showed improvement (PR or SD) was 1 patient out of 8 patients in the NAT2 Rapid group (12.5%) and 4 patients out of 8 patients (50%) in the NAT2 Non-Rapid (Slow and Intermediate) group in all 16 patients. In biliary tract cancer patients only (BDC: all 5 patients), 0 patients out of 1 patient in the NAT2 Rapid group (0%) and 3 patients out of 4 patients in the Non-Rapid group (75%) showed improvement (Table 4).

Regarding progression-free survival (PFS), a survival curve was created by the Kaplan-Meier method, and the median progression-free survival and its 95% confidence interval were calculated. As a result, the median was 37.0 days (CSR 11.4.1 Analysis of efficacy). Of these, the PFS median in the NAT2 Rapid group was 32 days, and that in the NAT2 Non-Rapid group was 58 days. By the way, in 4 patients out of 5 patients in the patients with the biliary tract cancer, the PFS median in the NAT2 Non-Rapid group was 99.5 days. In overall survival (OS), the median of all 16 patients was 3.5 months, of which 2.5 months in the NAT2 Rapid group and 6 months in the NAT2 Non-Rapid group (4 patients with biliary tract cancer). There was a significant difference between the two groups.

**(Table 4)**

| Patient type | Progression-free survival (median) | Overall survival (median) | Disease control rate | Tumor size change rate (average) |
|---|---|---|---|---|
| All patients (Number of patients = 16) | | | | |
| NAT2 Rapid (Number of patients = 8) | 32 days | 2.5 months | 12.5% | 23.0% |
| NAT2 Non-Rapid (Number of patients = 8) | 58 days | 6 months | 50.0% | 14.1% |
| Patient with biliary tract cancer (Number of patients = 5) | | | | |
| NAT2 Rapid (Number of patients = 1) | 22 days | 2 months | 0% | 5.3% |
| NAT2 Non-Rapid (Number of patients = 4) | 99.5 days | 6 months | 75.0% | -6.6% |

JPH203 is taken up by the liver and metabolized. JPH203 taken up by the liver is acetylated and converted to N-acetyl-JPH203 (Nac-JPH203), and Nac-JPH203 is excreted from the body depending on the blood flow velocity. Both JPH203 and Nac-JPH203 are taken up by OATP (organic anion transporter) into a hepatocyte, and excretion from the hepatocyte is performed by Mrp2 (multidrug resistance associated protein 2). Since the uptake rate of JPH203 (Nac-JPH203) by OATP1B1 is lower than the excretion rate by Mrp2, it is considered that the rate-determining step of the metabolism of JPH203 is the uptake process (rats). However, in a human, there is a large amount of stasis in the hepatocyte due to the relation with hepatic blood flow velocity, and it is thought that intracellular metabolism after uptake (process of acetylation by NAT2) is the rate-determining step.

The NAT2 of the subjects who participated in the phase I study was analyzed with 7 SNPs, and the phenotype of each subject's NAT2 (acetylation rate: Rapid, Intermediate, and Slow) regardless of the combination of 2-SNPs, 3-SNPs, and 4-SNPs showed consistent results (Table 3) .

Since the Nac-JPH203/JPH203 ratio of the 17 patients who participated in the Phase I study was almost correlated between blood and urine, it is presumed that JPH203 was metabolized in the liver and then excreted in the blood according to its concentration, and is passively excreted from the kidney into urine (one patient was excluded because the concentration in urine result was not available at the analysis stage). Of the 16 patients, all patients with a high Nac-JPH203/JPH203 ratio were NAT2 Rapid type patients. Among them, the top 4 patients with high Nac-JPH203 production ratio showed an increase in liver function test values, and 2 patients of them were judged to be DLT. The toxicity of the Nac-JPH203 metabolite is still largely unknown, but the fact that histological change to the liver, kidneys, etc. in a 4-week repeated administration study of JPH203 in a dog deficient in NAT2 compared to a rat and a human was insignificant compared to a rat is a support for the clinical results.

On the other hand, regarding efficacy, 1 patient of 8 patients in the NAT2 Rapid group (colon cancer) showed SD (12.5%), whereas 4 patients of 8 patients in the NAT2 Non-Rapid group showed PR or SD (50%). The overall survival was significantly longer in the NAT2 Non-Rapid group than in the NAT2 Rapid group, and median in progression-free survival (PFS) was also approximately twice as long in the NAT2 Non-Rapid group compared to the NAT2 Rapid group. Since 3 patients of the 4 patients suggesting the efficacy have biliary tract cancer, it was suggested that the efficacy of JPH203 may be expressed more effectively in the tissue in which metabolism by NAT2 gradually progresses and the local concentration of JPH203 is maintained above a certain level. The cases 102, 104, and 403 were eventually PD in the NAT2 Rapid group, but PFS was long in the NAT2 Rapid group. The ratio of Nac-JPH203/JPH203 in the blood of these cases was relatively low, and it is presumed that the safety and efficacy of JPH203 were superior in the NAT2 Rapid group.

Therefore, it was suggested that SNP analysis of NAT2 is useful as a "companion diagnostic" to identify an effective subject (patient with a cancer etc.) for JPH203 treatment and maximize their antitumor activity.

In addition, regarding the safety and efficacy of JPH203, the predominance of administration to the patient having the Non-Rapid type NAT2 gene can be further confirmed by a randomized comparative controlled study in which the target patients to be treated are classified into a group with a Non-Rapid type gene and a group with Rapid type gene in advance.

In such a study, JPH203 was administered, for example, at 25 mg/m², as one cycle of a total of 14 days with the drug withdrawal for 9 days following continuous administration for 5 days, and the investigational drug assigned to each subject is continuously administered intravenously over 90 minutes using a syringe pump or an infusion pump in total volume (100 mL).

As for the data analysis method, for example, in progression-free survival based on blinded independent central assessment, all subjects who received the investigational drug even once and for which efficacy data are available, for each NAT2 metabolism type (Rapid type, Non-Rapid type), the Cox proportional hazard model was used to calculate the hazard ratio and its 95% confidence interval for each population (Rapid type, Non-Rapid type). Furthermore, the p-value for the interaction can be calculated by the Cox proportional hazard model including the interaction of the administration group and the NAT2 metabolism type.

In addition, for the total survival time, for each NAT2 metabolism type (Rapid type, Non-Rapid type), the Cox proportional hazard model was used to calculate the hazard ratio and its 95% confidence interval for each population (Rapid type, Non-Rapid type). Furthermore, the p-value for the interaction is calculated by the Cox proportional hazard model including the interaction of the administration group and the NAT2 metabolism type. In addition, for the progression-free survival based on doctor's evaluation, for each NAT2 metabolism type (Rapid type, Non-Rapid type), regarding the progression-free survival (PFS) based on the evaluation of the doctor who is responsible for or shares the clinical trial, the Cox proportional hazard model was used to calculate the hazard ratio and its 95% confidence interval for each population (Rapid type, Non-Rapid type). Furthermore, the p-value for the interaction is calculated by the Cox proportional hazard model including the interaction of the administration group and the NAT2 metabolism type.

### Example 2

### Combination experiment of JPH203 and NAT2 inhibitor

Whether the concentration of JPH203 in the liver in plasma increases or the concentration of its metabolite Nac-JPH203 decreases is evaluated by administration of acetaminophen (APAP), which is a NAT2 inhibitor.

The animal used was a male SD rat 8 weeks old, and 1.20 nmol/(min/kg) of JPH203 was administered. JPH203 was diluted to the concentration with physiological saline and the rate of administration is 20 µL/min. Acetaminophen (500 and 1500 mg/kg) was administered 30 minutes before administration of JPH203. (0.5% aqueous methylcellulose solution). The time of blood collection was 0 (blank), 30, 60, 120, 180, and 240 minutes after administration of JPH203. The amount of sample to be collected was 0.2 mL for plasma and about 0.4 mL for blood, and the liver, kidney, brain and CSF were collected.

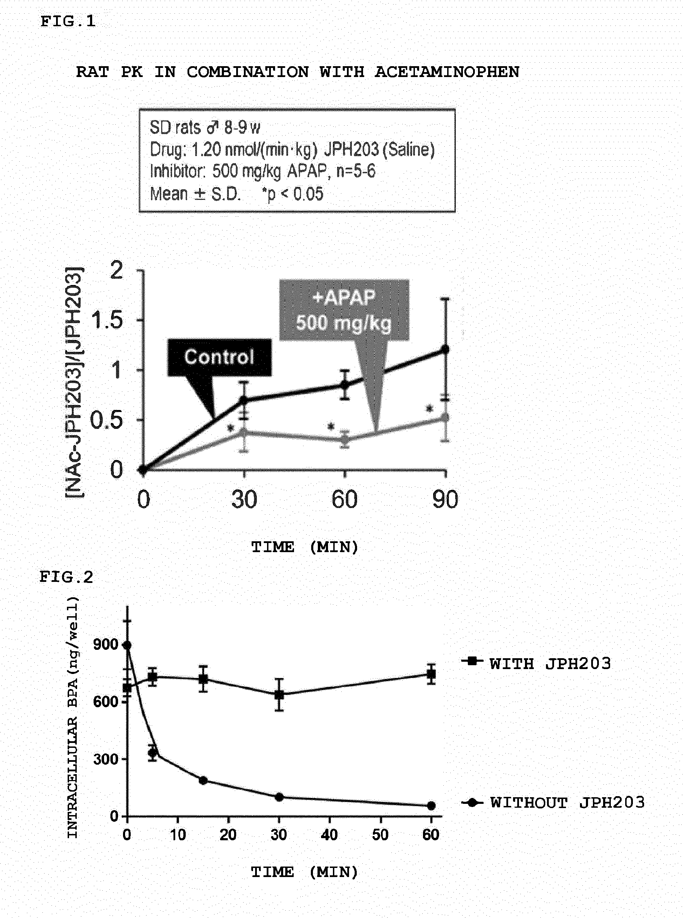

The results are shown in Fig. 1. The result shows that the combined use of JPH203 and the NAT2 inhibitor significantly reduces the ratio of Nac-JPH203/JPH203 in rat blood, slows the metabolic rate of JPH203, and eventually increases the blood concentration of JPH203, compared with the use of JPH203 alone, and the effect of JPH203 can be maintained.

### Example 3

A pancreatic cancer-derived cell T3M4 was used as a representative cancer cell, BPA was added to the cell, and 10 µM of JPH203 was added 15 minutes later, and the concentration of BPA contained in the cell was measured. The results are shown in Fig. 2. When JPH203 is not added (**•**), the intracellular concentration of BPA decreases as it is excreted from the cell over time. On the other hand, when JPH203 is added (■), it can be seen that the intracellular concentration is maintained because the excretion of BPA is suppressed by JPH203, and the difference in intracellular concentration between the two is about 10 times after 30 minutes.

### Industrial Applicability

The pharmaceutical composition of the present invention makes it possible to provide a treatment for a cancer or the like, and the pharmaceutical composition enhances the safety of the drug and improves the efficacy of the effect.

## Claims

1. A pharmaceutical composition comprising O-(5-amino-2-phenylbenzoxazole-7-yl)methyl-3,5-dichloro-L-tyrosine, or a pharmaceutically acceptable salt thereof, for use in treatment of a cancerous disease in a subject, the pharmaceutical composition being administered to the subject having a Non-Rapid (Slow and/or Intermediate) type NAT2 gene.

2. The pharmaceutical composition according to claim 1, wherein the cancerous disease is selected from the group consisting of biliary tract cancer, colon cancer, pancreatic cancer, esophageal cancer, breast cancer, lung cancer, prostate cancer, bladder cancer, brain tumor, stomach cancer, liver cancer, skin cancer, villous cancer, kidney cancer, head and neck cancer, tongue cancer, metastatic cancer, and invasive cancer.

3. The pharmaceutical composition according to claim 2, wherein the cancerous disease is selected from the group consisting of biliary tract cancer, colon cancer, pancreatic cancer, esophageal cancer, and breast cancer.

4. The pharmaceutical composition according to any one of claims 1 to 3, wherein the pharmaceutical composition is administered as one cycle with a drug withdrawal for a certain period following continuous administration for a certain period.

5. The pharmaceutical composition according to claim 4, wherein the pharmaceutical composition is administered as one cycle of a total of 14 days with the drug withdrawal for 9 days following continuous administration for 5 days.

6. The pharmaceutical composition according to any one of claims 1 to 5, wherein the pharmaceutical composition is administered to the subject at a dose of 1 to 60 mg/m².

7. The pharmaceutical composition according to claim 6, wherein the pharmaceutical composition is administered to the subject at a dose of 12.5 to 60 mg/m².

8. The pharmaceutical composition according to claim 7, wherein the pharmaceutical composition is administered to the subject at a dose of 12.5 to 25 mg/m².

9. The pharmaceutical composition according to any one of claims 1 to 8, wherein a certain amount of the pharmaceutical composition is continuously administered intravenously over a certain period of time.

10. The pharmaceutical composition according to claim 9, wherein 100 mL of the pharmaceutical composition is continuously administered intravenously over 90 minutes.

11. A method for diagnosing or predicting whether O-(5-amino-2-phenylbenzoxazole-7-yl)methyl-3,5-dichloro-L-tyrosine is effective for a specific cancerous disease, the method comprising a step of determining whether or not NAT2 gene in a subject has a Non-Rapid type NAT2 gene.

12. A genetic diagnosis kit for determining whether or not NAT2 gene in a subject has a Non-Rapid type NAT2 gene in order to diagnose whether O-(5-amino-2-phenylbenzoxazole-7-yl)methyl-3,5-dichloro-L-tyrosine is effective for a specific cancerous disease.

13. A pharmaceutical composition comprising O-(5-amino-2-phenylbenzoxazole-7-yl)methyl-3,5-dichloro-L-tyrosine or a pharmaceutically acceptable salt thereof for use in combination with the genetic diagnosis kit according to claim 12.

14. A pharmaceutical composition comprising O-(5-amino-2-phenylbenzoxazole-7-yl)methyl-3,5-dichloro-L-tyrosine or a pharmaceutically acceptable salt thereof for treating cancer, wherein the pharmaceutical composition is used in combination with NAT2 genetic diagnosis.

15. A pharmaceutical composition comprising O-(5-amino-2-phenylbenzoxazole-7-yl)methyl-3,5-dichloro-L-tyrosine, or a pharmaceutically acceptable salt thereof, for use in treatment of a cancerous disease in a subject, wherein the pharmaceutical composition is used in combination with a NAT2 inhibitor.

16. The pharmaceutical composition according to claim 15, wherein the NAT2 inhibitor is acetaminophen.

17. The pharmaceutical composition according to claim 15, wherein the pharmaceutical composition is administered to the subject having the Rapid type NAT2 gene.

18. A pharmaceutical composition comprising O-(5-amino-2-phenylbenzoxazole-7-yl)methyl-3,5-dichloro-L-tyrosine, or a pharmaceutically acceptable salt thereof, for use in treatment of a cancer in a subject, wherein the pharmaceutical composition is administered under the following regimens:
(1) the subject having the Non-Rapid (Slow and/or Intermediate) type NAT2 gene is identified and selected;
(2) boron-containing compound is administered to the subject identified as having the Non-Rapid (Slow and/or Intermediate) type NAT2 gene prior to administration of the pharmaceutical composition; and
(3) the pharmaceutical composition is administered to the subject.

19. The pharmaceutical composition according to any one of claims 1 to 10 and 13 to 17, wherein the cancer is further killed by a neutron capture therapy (BNCT) after administration of the pharmaceutical composition.

20. The pharmaceutical composition according to any one of claims 1 to 10 and 13 to 19, the method according to claim 11, or the kit according to claim 12, wherein the subject is a human.
